# EUROPEAN PATENT APPLICATION

(11) **EP 4 675 258 A1**
(43) Date of publication of application: **07.01.2026**
(21) Application number: 24185972.7
(22) Date of filing: 02.07.2024
(51) Int. Cl.: G01N 21/25, G01B 11/06, G01N 21/31, G01N 21/3563, G01N 21/359, G01J 3/46, G01N 21/89, G01N 33/36, G01N 33/44, G01N 21/84, G01N 21/85, G01N 1/28

(54) **QUALITY CONTROL OF TEXTILE, LEATHER, AND/OR FOOTWEAR FEED MATERIALS**

(71) Applicant: Tomra Horizon AS, 1385 Asker (NO)
(72) Inventor: Becker, Thilo, Asker (NO); Althaus, Malte, Berlin (DE); Stemberger, Johanna, Asker (NO)
(74) Representative: AWA Sweden AB

(57) **Abstract**

The present disclosure relates to a method of quality control of textiles and more generally textile, leather, and/or footwear feed material, TLF-feed material, the method comprising: providing a material batch (MB) as a material batch layer (MB-L) in a transporting arrangement (100), wherein the material batch layer (MB-L) comprises fragmented TLF-feed material of one or more material compositions, wherein any individual pieces of at least a main portion of the fragmented TLF-feed material have a footprint area of at most 500 cm2; transporting, by means of the transport arrangement (100), said material batch layer (MB-L) to a detection zone (d-z) of a sensor system (300); providing, by means of the sensor system (300), sensor data of the material batch layer (MB-L) in the detection zone (d-z); determining, based on said sensor data, fraction data indicative of at least a first characteristic of the fragmented TLF-feed material in the material batch layer (MB-L), wherein said at least a first characteristic includes material composition and/or average color and/or color distribution. An arrangement is also disclosed.

## Description

### Technical Field

The present disclosure generally relates to a method of quality control of textile, leather, and/or footwear feed materials, TLF-feed materials, and an arrangement for quality control of the same.

### Background of the Invention

Textile waste disposal is a key issue around the world. Currently, the primary end destination of textile waste are incineration and landfills, which have detrimental effects on both people and the environment. These practices contribute to pollution, resource depletion, and greenhouse gas emissions. This linear disposal perpetuates increasing amounts of waste rather than promotes sustainability. Thus, there is a need for proper and efficient textile waste processing.

In certain applications, such as processing textile waste including unsorted textiles, it is necessary to classify textile waste before sorting the textile waste accordingly based on classification. In recent years, effort has been made to develop improved systems and processes. Moreover, there have been efforts to mitigate negative environmental effects of textile waste processing. Another aspect is related to how to determine characteristics, such as fiber composition, of textile material. The following patent applications are a few examples relating to above-mentioned aspects.

US2022214273 AA relates to determining a fiber composition of a textile sample. The sample is irradiated with near infrared, NIR, light. Reflected NIR light from the sample is captured. A vector of spectral values is determined based on the captured light. The vector is input to a deep neural network, DNN. The DNN comprises a sequence of layers of nodes, in particular an input layer, at least two intermediate layers, and an output layer. The nodes of successive layers of the sequence are interconnected via weighted edges. The DNN outputs for each of a plurality of fiber material types a numerical relative composition amount.

WO22195168 A1 relates to a method and arrangement for processing and measuring textile waste. Textile waste comprising at least a first component of organic fibers and a second component of non-organic fibers is processed in a processing stage. A suspension sample is obtained from the output of the processing stage. One or more color components are fed to the sample which is mixed for a predetermined period. The suspension sample is directed to a measurement chamber where optical radiation is directed at the suspension. Interaction of the optical radiation with the suspension is detected optically and the amount of different components in the sample is determined based on the detection.

When processing textile waste in processing plants, it is desirable to process textile waste at a throughput as high as possible. Processing textile waste requires a solution to reliably determine characteristics of textile waste, such as fiber composition, which is difficult to do since unsorted textile waste typically comprises a vast amount of different textile items, varying greatly in textile composition, color, and size. Moreover, textile items in the textile waste may randomly obscure one another or be randomly folded in some manner, thus making any measurement less reliable.

Thus, there is a need for an improved arrangement and method for quality control of textile, leather, and/or footwear feed materials.

### Summary of the Invention

In view of the above, it is an object of the present disclosure to provide an improved method of quality control of textile, leather, and/or footwear feed material, TLF-feed material. It is also an object of the present disclosure to provide an improved arrangement for quality control of the same. Moreover, it is an object to provide an arrangement and method at least facilitating estimation of quantity of one or more constituents of textile material. Further, it is an object to provide a cost-effective arrangement and method for quality control of TLF-feed material, such as from TLF-feed material sorting. In addition, it is an object of the invention to provide an arrangement and method for quality control of the same, which arrangement and method enables modularity and facilitates scalability.

To achieve at least one of the above objects, and also other objects that will be evident from the following description, an arrangement and a method is provided, which arrangement and method is suitable for sorting textile, leather, and/or footwear feed material, TLF-feed material, and optionally only a specific subgroup thereof. The terms "textile", "leather", "footwear" and "feed material" are defined in a subsequent section of this disclosure, see "Feed material and related information".

### Introductory disclosure

The present disclosure is based on the inventors' realization that quality control of TLF-feed material may be determined more reliably and faster by providing a material batch as a material batch layer in a transporting arrangement, wherein the material batch layer comprises fragmented TLF-feed material of one or more material compositions, wherein any individual pieces of at least a main portion of the fragmented TLF-feed material have a footprint area of at most 500 cm². By this, a more even and optionally a more homogeneous material flow is provided. Further, it may reduce the risk of any pieces of items in the material batch layer randomly obscuring one another or being randomly folded in some manner. Moreover, smaller pieces may result in a better statistical distribution. A sensor system may therefore provide more reliable sensor data of the material batch layer, whereby a method and arrangement may advantageously provide more reliable quality control of TLF-feed material or of any particular sub-group thereof.

In the context of the application, quality control may refer to determining and/or monitoring one or more material constituents of textile material or more generally TLF-feed material, in terms of fractions and/or quantities and/or to controlling whether at least a first portion of TLF-feed material has a certain quality represented by one or more fractions of material constituents.

The term "throughput" may refer to a capacity of the amount of feed material that can be received and/or sorted in a given amount of time. The term "throughput" may refer to the capacity of feed material an arrangement can receive and/or sort, actively and passively, in a given amount of time. As a non-limiting example, throughput may be quantified in tons per hour, tons per day, tons per week, tons per month, and/or tons per year. As a non-limiting example, a high throughput may refer to at least 2 tons per hour, at least 2.5 tons per hour, 3 tons per hour, at least 3.5 tons per hour, at least 4 tons per hour, or at least 4.5 tons per hour.

By at least one material batch, it refers to one material batch or a plurality of material batches at least comprising or containing feed material such as TLF-feed material. Further, material batch refers to a batch of feed material that is to be sorted. A material batch may have a limited quantity of feed material. For instance, a material batch may be provided e.g., by means of a container, a plastic bale, or may be provided from a warehouse for storing a material batch or a plurality of material batches. Moreover, sorting at least one material batch refers to sorting the feed material of the material batch, which generally is provided in form of whole items, damaged items, fragmented items and/or feed material, or the like. A plurality of material batches may be provided in a continuous stream. The method and arrangement of the present disclosure may be adapted for processing a continuous stream of material batches.

The feed material of said at least one material batch includes TLF-feed material (see next section). The feed material of said at least one material batch may also include non-textiles, such as metal, plastics, and other hazardous/non-hazardous substances, associated with various textile items (prints, coatings, stains, etc.). The feed material of said at least one material batch may also include non-textiles, such as metal, plastics, and other hazardous/non-hazardous substances, which is not associated with textile items (other waste material or unsorted feed material in general). As a non-limiting example, the feed material of the material batch may include general waste. As a non-limiting example, the feed material of the material batch may be unsorted feed material including TLF-feed material. As a non-limiting example, the feed material of the material batch may include textile waste including TLF-feed material. As a non-limiting example, said at least one material batch may comprise at least 50%, or at least 80%, TLF-feed material by weight or by volume. As a non-limiting example, said at least one material batch may contain only TLF-feed material.

### Feed material and related information

Admittedly, today there are various different terms and abbreviations for denoting textile material and materials which are, depending on context, associated with textile material. For instance, the European Commission group the following into the same ECO system: Textile, Clothing, Leather and Fur and sometimes Textile, Clothing, Leather and Footwear.

In the context of this application, the term TLF-feed material refers to textile, leather, and/or footwear feed material, wherein textile feed material includes fur as discussed in more detail below.

In the context of this application, feed material refers to material being received by the arrangement for processing. Processing may include events such as detection, classification, transportation, and/or sorting.

ASTM Standard D123 - 23, henceforth referred to as "The ASTM Standard", is a standard including a compilation of all terminology developed by Committee D13 on Textiles. This international standard was developed in accordance with internationally recognized principles on standardization established in the Decision on Principles for the Development of International Standards, Guides and Recommendations issued by the World Trade Organization Technical Barriers to Trade (TBT) Committee. The ASTM Standard is incorporated herein by reference.

Terms of the present disclosure are used with a meaning consistent with the ASTM Standard, a few examples being: "textile", "textile fiber", "fabric, "garment", "leather".

Although broad definitions are applied throughout, a few specific examples are mentioned in the following. As non-limiting examples, fiber-based materials referred to herein include fibers, yarns, filaments, threads, different fabric types such as woven and non-woven fabrics, cloth, leather etc. As non-limiting examples, fiber-based materials may include natural fibers, man-made fibers, or a combination thereof.

Moreover, non-limiting examples of natural fibers include fibers of vegetable origin, such as cotton or flax/linen, and/or of animal origin, such as wool. Non-limiting examples of man-made fibers include natural polymers from the various kingdoms of life, such as lyocell, modal and viscose from the plantae/vegetable kingdom, synthetic polymers fossil-based or bio-based, such as polyester, or inorganic fibers, such as glass and metal fibers.

In the context of this application, the term "textile item" refers to an item containing at least some textile fiber, for instance containing at least 20% or at least 40% or at least 60% or at least 80% by weight of textile fibers. As a non-limiting example, textile items may include textile products. Textile products may refer to those products comprising at least 80% by weight of textile fibers. Textile items may comprise 1 %-10%, 10%-20%, 20%-30%, 30%-40%, 40%-50%, 50%-60%, 60%-70%, 70%-80%, 80%-90%, 90%-99%, 100%, by weight of textile fibers, or any combination of the listed end numbers, in a continuous interval or in two or more separate intervals.

In addition to garments, the term "textile item" may include other items such as bags and/or clothing accessories, household items such as towels, tablecloths, curtains, rugs, bedlinen, pillows, duvets, and upholstery textiles, as well as other types of items such as technical textiles.

As mentioned, TLF-feed material may include footwear. Non-limiting examples of footwear includes shoes, boots, or other outer coverings for the feet. Footwear may be at least partly manufactured from leather, textiles, synthetics, rubber, foam, and/or plastic.

The following is a non-exhaustive list of some textile compositions: cashmere, cotton, linen, polyester, synthetic, silk, stretch, viscose, wool, elastane. A textile item may comprise a mono-material or a blend of materials. As a non-limiting example, a first textile composition is a polyester blend having the composition 42% polyester, 45% cotton, 13% viscose. As a non-limiting example, a second textile composition has the composition 100% polyester. As a non-limiting example, a third textile composition has a composition of 100% cotton. As a non-limiting example, a fourth textile composition is a linen blend having the composition 55% linen, 45% viscose. As a non-limiting example, a fifth textile composition is a cotton blend having the composition 62% cotton, 36% viscose, 2% elastane.

The present disclosure is not limited to any particular TLF feed material or composition thereof. For instance, a textile item may comprise a TLF feed material at any fraction between 0% and 100%, depending on any other eventually comprised TLF feed materials. A first TLF composition may e.g. have the composition 10 % leather and 90 % linen blend. A second TLF composition may e.g. have the composition 98 % fur and 2 % cotton. A textile item may include or be composed of any textile composition mentioned herein and/or any other textile composition not disclosed herein but known in the field of textiles. A textile or fabric may comprise a blend or patchwork of any two or more textile compositions, or a blend or patchwork of any two or more TLF compositions.

Products of TLF feed material, most notably technical textiles, are present across the economic sectors, such as in agriculture (agro textiles), construction (notably geotextiles but also others, such as insulation and flooring products), the transport, defense and space industries and medical products, and include products meant for both professional and non-professional use, such as filters, cleaning products, hygiene products, protective equipment and certain sports and work wear. Smart textiles, i.e. textiles that are able to sense and respond to changes in their environment (including the wearer's conditions), are an area of particular growth and technical development for technical textiles. There is one category of smart textiles (called "e-textiles") that contain electronic components.

A non-exhaustive list of textile structure includes: fabrics from the woven, braided, knitted groups of textiles, for example: boucle, tweed, corduroy, brocade, fine suiting, jacquard, lace, embroidery, plain, shirting, tartan, uncut or cut velvet. Textile items may be provided with prints and/or coatings.

In the context of the application, the term "TLF fragment" refers to items of TLF feed material, which have been fragmented and thus reduced in size. The size reduction can occur, for example, through cutting, shredding and/or tearing. TLF fragments may include foreign non-textile objects such as zippers, buttons and/or prints. One particular form of TLF fragments include so called textile shoddy, e.g., torn textile fabrics used for the production of non-woven textiles and/or re-spinning into recycled yarn. TLF fragments may include and/or refer to cut and/or shredded and/or torn and/or any other forms of remains of said items and/or feed material. As a non-limiting example, TLF fragments may include clearly distinguishable pieces of items and/or feed material having substantially no clearly distinguishable form, such as shoddy and/or textile fluff. The arrangement and/or the method detailed in the present disclosure may equally be applied for sorting TLF fragments.

### Method of quality control of TLF-feed material

According to a first aspect of the disclosure, a method of quality control of TLF-feed material. The method comprises: providing at least one material batch as a material batch layer in a transporting arrangement, wherein the material batch layer comprises fragmented TLF-feed material of one or more material compositions, wherein any individual pieces of at least a main portion of the fragmented TLF-feed material have a footprint area of at most 500 cm2; transporting, by means of the transport arrangement, said material batch layer to a detection zone; providing, by means of a sensor system, sensor data of the material batch layer in the detection zone; determining, based on said sensor data, fraction data indicative of at least a first characteristic of the fragmented TLF-feed material in the material batch layer, wherein said at least a first characteristic includes material composition and/or average color and/or color distribution.

By providing fragmented TLF-feed material as a material batch layer, a more even and optionally homogenous material flow is provided. Further, it reduces the risk of any pieces in the material batch layer randomly obscuring one another or being randomly folded in some manner. Thereby, the sensor system may provide more reliable sensor data of the material batch layer so that the at least a first characteristic of TLF-feed material in the material batch more be determined more accurately. Moreover, the method advantageously enables continuous quality control monitoring. Moreover, the method advantageously facilitates continuous monitoring of at least a first characteristic of TLF-feed material in the material batch. Thus, the method may advantageously provide more accurate quality control, since it is based on more data regarding the TLF-feed material in the material batch and is not extrapolated from a sample of the TLF-feed material in the material batch pre- or post-processing the material batch.

Moreover, said at least a first characteristic of TLF-feed material of the fragmented TLF-feed material may include: TLF-feed material composition, and/or TLF-feed material composition fractions, and/or colors, and/or sizes, and/or portion sizes, and/or textile structures, and/or textile product types, and/or moisture content, and/or hazardous non-textile substances, and/or non-hazardous non-textile substances, textile print substances, and/or surface coating substances.

According to one embodiment, said step of providing at least one material batch as a material batch layer in a transporting arrangement further comprises: providing one or more items of TLF-feed material; dividing, by means of a divider arrangement, the items into fragmented TLF-feed material before said material batch is provided as a material batch layer, wherein, optionally, said step of dividing the items into fragmented TLF-feed material includes cutting and/or shredding and/or tearing the items.

Following the step of dividing the items into fragmented TLF-feed material, the fragmented TLF-feed material is transported to the detection zone. The fragmented TLF-feed material may be transported by a plurality of conveyor belt segments and/or free fall arrangement segments which cooperatively and successively spread the fragmented TLF-feed material of the material batch so as to provide the material batch as a material batch layer comprising fragmented TLF-feed material. Alternatively, or in combination, following the step of dividing the TLF-feed material items into fragmented TLF-feed material, the fragmented TLF-feed material may be transported to a spreading arrangement to provide the material batch as a material batch layer comprising the fragmented TLF-feed material. The spreading arrangement may include a stationary spreading element and/or a moveable spreading element arranged to spread the material batch substantially across a width of a conveyor belt or a free-falling zone. The material batch layer may be provided so as to have a layer thickness of a certain layer thickness. Said certain layer thickness may be adjustable by means of adjusting transport speed and/or moving the spreading element if moveably arranged. By dividing TLF-feed material items to provide fragmented TLF-feed material, the material batch may be provided with a more homogenous and continuous form.

Following the step of dividing the TLF-feed material items into fragmented TLF-feed material, the fragmented TLF-feed material may be subjected to size sorting to separate oversized pieces of fragmented TLF-feed material from the material batch before the material batch is provided as a material batch layer comprising fragmented TLF-feed material to the detection zone. By this, it may further reduce an amount of oversized TLF-feed material pieces of fragmented TLF-feed material. Thus, the material batch layer may become more homogenous, whereby sensor data thereof is advantageously more reliable. As a non-limiting example, said size sorting may be carried out by means of a drum screen. As a non-limiting example, a drum screen may be used to separate TLF-feed material pieces having a cross-sectional extension larger than a predefined cross-section extension. By separating the oversized material, the resulting material batch stream may be more stable, thereby resulting in a decreased need of personnel for unclogging the arrangement.

However, the material batch may be subjected to size sorting by other means so as to reduce an amount of oversized TLF-feed material pieces, for instance, by means of a classification and sorting arrangement configured to classify oversized TLF-feed material pieces in the fragmented TLF-feed material as oversized and sort the oversized TLF-feed material pieces from the material batch accordingly. Oversized TLF-feed material pieces may be transported to a divider arrangement downstream or the same divider arrangement to further divide the oversized TLF-feed material pieces to acceptable sizes.

According to one embodiment, the method is adapted to provide a continuous stream of matter including fragmented TLF-feed material. A continuous stream of matter may be provided continuously or by providing each material batch of the at least one material batch in a sequential manner.

According to one embodiment, the arrangement may further comprise a pre-collection arrangement. The pre-collection arrangement may be adapted to collect at least one material batch of matter including TLF-feed material whereafter the matter including TLF-feed material is provided as a continuous stream of matter. The pre-collection arrangement may be configured to receive at least one material batch including fragmented TLF-feed material to increase a bulk density of fragmented TLF-feed material. By increasing bulk density, the material batch including the fragmented TLF-feed material may at least partially be made more compact. An advantage of this is a decreased need of storage capacity of the arrangement and the increased quantity that may be transported and/or processed by the arrangement. The pre-collection arrangement may be adapted to collect at least one material batch of matter including fragmented TLF-feed material whereafter the matter including TLF-feed material is provided as a continuous stream of matter of fragmented TLF-feed material.

According to one embodiment, said fraction data indicates at least a second characteristic of the fragmented TLF-feed material in the material batch layer, wherein said at least a second characteristic includes material composition and/or average color and/or color distribution.

By this, a plurality of characteristics may be continuously monitored. As a non-limiting example, the first characteristic may be an amount of a first TLF-feed material constituent, such as cotton, and a second characteristic may be an amount of a second TLF-feed material constituent, such as polyester. Thus, the method may advantageously determine fraction data indicative of said amounts of example TLF-feed material constituents of cotton and polyester. The fraction data may enable outputting of quality data indicating a respective weight and/or WT% and/or V/V% for cotton and polyester. Thus, material composition of the material batch may be continuously determined, thus facilitating a step of determining how much TLF-feed material has been identified in the material batch and further providing a more reliable estimate than a sample measurement made pre- or post-processing.

According to one embodiment, the material batch layer is at least partially transported by means of a conveyor arrangement, wherein said step of transporting, by means of the transport arrangement, said material batch layer to a detection zone of a sensor system includes: transporting the material batch layer substantially across a width of a conveyor belt of the conveyor arrangement.

The conveyor arrangement may be configured to transport said material batch from an arrangement input or a divider arrangement to a detection zone of the sensor system as said material batch layer comprising fragmented TLF-feed material. The conveyor arrangement may be configured to transport said material batch layer including fragmented TLF-feed material from the detection zone of the sensor system and arrangement output. The transportation arrangement may comprise one or more transportation segments for transporting material batch and/or material batch layer including fragmented TLF-feed material between two arrangement locations. The conveyor arrangement may comprise one or more conveyor belt segments. The conveyor arrangement may be configurable so as to vary transport speed of one or more conveyor belt segments. As a non-limiting example, any of the conveyor belt segments may be adapted with a width so as to achieve a certain throughput of the arrangement. By having a wider conveyor belt segment at the detection zone of the sensor system, the material batch layer may be distributed over a larger width, thereby reducing the thickness of the material batch layer and further reducing the risk of overlapping of different TLF-feed material, whereby accuracy of sensor data are improved. Alternatively, or in combination, a wider conveyor belt segment at the detection zone of the sensor system advantageously enables an increased throughput of the arrangement, since more material can be provided by the material batch layer when provided at particular layer thickness. As non-limiting examples, any conveyor belt segment may be adapted so as to have a width in an interval of 0.5-1 m, 1-1.5 m, 1.5-2 m, 2-2.5 m, 2.5-3 m, 3-3.5 m, 3.5-4 m, 4-4.5 m, 4.5-5 m, or more than 5 m, or in an interval formed by any combination thereof.

According to one embodiment, the material batch layer is at least partially transported in a free-falling arrangement, wherein said step of transporting, by means of the transport arrangement, said material batch layer to a detection zone of a sensor system includes: transporting the material batch layer substantially across a width of a free-falling zone of the free-falling arrangement. The free-falling zone of the free-falling arrangement may be provided by a free-falling arrangement segment of the free-falling arrangement. As non-limiting examples, the free-falling arrangement segment may be a drop from a first conveyor belt segment to a second conveyor belt segment or a chute guiding material batch and/or material batch layer from a first conveyor belt segment to a second conveyor belt segment. The sensor system may be arranged so that the detection zone and the free-falling zone at least partly overlap or coincide. By having a wider free-falling segment at the detection zone of the sensor system, the material batch layer may be distributed over a larger width, thereby reducing the thickness of the material batch layer and further reducing the risk of overlapping of different TLF-feed material, whereby accuracy of sensor data are improved. Alternatively, or in combination, a wider free-falling arrangement segment at the detection zone of the sensor system advantageously enables an increased throughput of the arrangement, since more material can be provided by the material batch layer when provided at particular layer thickness. As non-limiting examples, any free-falling arrangement segment may be adapted so as to have a width in an interval of 0.5-1 m, 1-1.5 m, 1.5-2 m, 2-2.5 m, 2.5-3 m, 3-3.5 m, 3.5-4 m, 4-4.5 m, 4.5-5 m, or more than 5 m, or in an interval formed by any combination thereof.

According to one embodiment, said step of providing, by means of the sensor system, sensor data of the material batch layer includes: providing, by means of a spectroscopy system, spectroscopy sensor data of the material batch layer, wherein the spectroscopy system is preferably a near-infrared, NIR, spectroscopy system.

The method may, by means of a spectroscopy system, such as a NIR spectroscopy system, advantageously provide more accurate sensor data of the TLF-feed material in the material batch layer. In particular, NIR spectroscopy system may advantageously provide more accurate sensor data. The accuracy thereof is further improved by providing the material batch layer with a layer thickness so that the matter can be treated approximately homogeneous in a depth direction, i.e., a TLF-feed material composition at a surface level of the material batch layer is statistically a satisfactory representation of a TLF-feed material composition at depth. For instance, a TLF-feed material composition at a surface level of the material batch layer is on average X% substantially equal to a TLF-feed material composition at depth, wherein X is at least 50, 60, 70, 80, 90, 95, or 99. Preferable, X should be as high as possible for any given material batch layer and selected thickness thereof. Generally, X increases with a decreased layer thickness of the material batch layer. In other words, sensor data may be more representative and reliable for the material batch layer if reducing the layer thickness further so that X can be increased. However, with a reduced layer thickness, the throughput is reduced for any given layer width. The choice of layer thickness may be selected depending on the composition of the TLF-feed material. As a non-limiting example, the layer thickness may be in an interval of 10 mm to 250 mm. As a non-limiting example, the layer thickness may be in any interval of 10 mm to 50 mm, 50 mm to 100 mm, 100 mm to 150 mm, 150 mm to 200 mm, 200 mm to 250 mm.

The spectroscopy system may be configured to provide a spectrum of said fragmented TLF-feed material within the detection zone. By such a spectroscopy system, the arrangement may advantageously enable accurate sensor data of fragmented TLF-feed material, which in turn improves accuracy of said characteristics of TLF-feed material determined by the method. Spectroscopy system may include near-infrared, NIR, spectroscopy system. NIR spectroscopy may be advantageously enable detection of characteristics sub-surface. NIR may refer to e.g., near-infrared region of the electromagnetic spectrum. As a non-limiting example, near-infrared region of the electromagnetic spectrum may be in the interval of 780 nm to 2500 nm. As an alternative, or in combination, such a spectroscopy system may be configured to use region of the electromagnetic spectrum outside NIR, such as the visible region of electromagnetic spectrum. Moreover, as an alternative, or in combination, the spectroscopy system may be configured to use region of x-ray spectrum. By this, more accurate material density measurements may be provided. The spectroscopy system may be configured to use both NIR spectroscopy and X-ray spectroscopy. The spectroscopy system may be a VIS/NIR spectroscopy system configured to detect visible spectrum and/or near-infrared spectrum.

The spectroscopy system may be configured to analyse light in the wavelength interval 400 - 1000 nm. The spectroscopy system may be configured to analyse light in the wavelength interval 500 - 1000 nm. The spectrometer may be configured to analyse light in the wavelength interval 1000 - 1900 nm. The spectroscopy system may be configured to analyse light having a wavelength above 900 nm. The spectroscopy system may be configured to analyse light in the wavelength interval 1900 - 2500 nm. The spectroscopy system may be configured to analyse light in the wavelength interval 2700 - 5300 nm. The spectroscopy system may be configured to analyse light in the wavelength interval 900 - 1700 nm. The spectroscopy system may be configured to analyse light in the wavelength interval 700 - 1400 nm. The spectroscopy system may analyse visible light. The spectroscopy system may analyse NIR light. The spectroscopy system may analyse IR light. Different types of spectroscopy system may be used depending on characteristics of the matter to be detected.

According to one embodiment, said step of providing, by means of the sensor system, sensor data of the material batch layer includes: providing, by means of the optical sensor system, optical sensor data of the material batch layer, wherein, optionally, the optical sensor system is configured with an artificial neural network configured to detect said at least first characteristic of fragmented TLF-feed material in the material batch based on images or image data of the optical sensor data acquired by the optical sensor system.

The optical sensor system may be configured to receive and analyze light reflected and/or scattered by the material batch layer in the detection zone through which the material batch layer comprising fragmented TLF-feed material is provided. The arrangement may be configured to determine characteristics of said fragmented TLF-feed material based on an analysis provided by the optical sensor arrangement.

The method may thereby advantageously facilitate the step of determining at least a first characteristic of fragmented TLF-feed material which cannot be detected, reliably or at all, by means of a spectroscopy system, such as a NIR spectroscopy system. Thereby, a greater variety of characteristics of fragmented TLF-feed material can be determined.

As a non-limiting example, the optical sensor system may comprise one or more RGB cameras and/or one or more RGB-NIR cameras. As a non-limiting examples, the optical sensor arrangement may include a digital image sensor such as charge-coupled device (CCD) or an active-pixel sensor (CMOS sensor). As a non-limiting example, the method includes a step of illuminating fragmented TLF-feed material in the detection zone by means of laser from a laser triangulation system, wherein the received light of the optical sensor system originates from the laser triangulation system.

According to one embodiment, the material batch comprises at least 70 WT% or 70 V/V% fragmented TLF-feed material, optionally the material batch comprises fragmented TLF-feed material in an interval 70-80 WT% or V/V%, 80-90 WT% or V/V%, 90-99 WT% or V/V%, or 100 WT% or V/V%. The material batch may comprises fragmented TLF-feed material in an interval of 10-20 WT% or V/V%, 20-30 WT% or V/V%, 30-40 WT% or V/V%, 40-50 WT% or V/V%, 50-60 WT% or V/V%, 60-70 WT% or V/V%, 70-80 WT% or V/V%, 80-90 WT% or V/V%, 90-99 WT% or V/V%, 99-100 WT% or V/V% or in any interval formed by any combination thereof. The material batch layer as provided may have an increased WT% or V/V% relative the material batch following one or more sorting steps by a sorting arrangement.

According to one embodiment, the material batch layer includes one or more material compositions at least partially or wholly selected from a group comprising: cotton, polyester, polyamides, acrylics, viscose, wool, silk, elastane, polypropylene, polyurethane, metals, acetate, cellulosic fibers, and/or combinations thereof. The material batch layer may include other materials, such as non-TLF-feed material materials, also.

The method may comprise a step of sorting out, by means of a magnet arrangement configured to attract ferrous metal, ferrous metal from said material batch or said material batch layer originating from the material batch. The method may comprise a step of sorting out, by means of an eddy current separator configured to remove non-ferrous metals, non-ferrous metals from said material batch or said material batch layer originating from the material batch. The method may thereby advantageously remove ferrous and/or non-ferrous metals from the material batch and/or the material batch layer comprising fragmented TLF-feed material. Since metal is valuable, profitability of the arrangement may be further increased by sorting out the metals. Sorting metal out from the material batch and/or material batch layer comprising fragmented TLF-feed material may result in a decrease of batch material downstream in the material batch and/or material batch layer, thereby further facilitating determining at least a first characteristic of TLF-feed material downstream.

According to one embodiment, said step of transporting, by means of the transport arrangement, said material batch layer to a detection zone of a sensor system comprises: providing the material batch layer to the detection zone at a transport speed selected from a transport speed interval of 0.1 to 20 meters per second, preferably 2 to 5 meters per second. The transport speed may be selected from a transport speed interval of 0.1-0.2 meters per second, 0.2-0.3 meters per second, 0.3-0.4 meters per second, 0.4-0.5 meters per second, 0.5-0.6 meters per second, 0.6-0.7 meters per second, 0.7-0.8 meters per second, 0.8-0.9 meters per second, 0.9-1 meters per second, 1-2 meters per second, 2-3 meters per second, 3-4 meters per second, 4-5 meters per second, 5-6 meters per second, 6-7 meters per second, 7-8 meters per second, 8-9 meters per second, 9-10 meters per second, 10-11 meters per second, 11-12 meters per second, 12-13 meters per second, 13-14 meters per second, 14-15 meters per second, 15-16 meters per second, 16-17 meters per second, 17-18 meters per second, 18-19 meters per second, 19-20 meters per second. The choice of transport speed of one or more conveyor belt segments may be selected depending on a width of said conveyor belt segment so as to enable a certain throughput and/or layer thickness of said material batch layer.

According to one embodiment, the method comprises: measuring a layer thickness of the material batch layer by means of a laser triangulation system. By this, the arrangement may correlate quality control data and material batch layer. If quality control data do not satisfy one or more conditions, the arrangement may be controlled so as to adjust the material batch layer as provided to a more preferred layer thickness which results in improved quality control data.

According to one embodiment, the method comprises: outputting quality data including at least a minimum and/or a maximum and/or an average and/or a median of one of the following based on said fraction data: a respective weight; a respective WT%; a respective V/V%, for each of said at least a first characteristic of the fragmented TLF-feed material, wherein, optionally, said quality data is displayed on a display device and/or provided to a computer-readable storage medium. This facilitates continuous monitoring of characteristics of one or more TLF-feed material materials of the fragmented TLF-feed materials. The arrangement may be configured to adjust processing of the material batch and/or material batch layer depending on quality control data.

According to one embodiment, the method comprises: estimating weight percentages and/or volume percentages of fragmented TLF-feed material of at least one of said at least first characteristic based on said fraction data and respective sizes of the one or more local surface areas of the material batch layer corresponding to said fraction data, and/or estimating weight and/or volume of fragmented TLF-feed material of at least one of said at least first characteristic based on said fraction data and respective sizes of the one or more local surface areas of the material batch layer associated with said fraction data, layer thickness of said one or more local surface areas of the material batch layer, and densities of said fragmented TLF-feed material of at least one of said at least first characteristic. By this, the method enables determining of amounts in terms of weight percentage and/or volume percentage of said TLF-feed material materials in a continuous manner during quality control monitoring.

### Arrangement for quality control of TLF-feed material

According to a second aspect of the disclosure, an arrangement for quality control of TLF-feed material is provided. The arrangement comprises: a transporting arrangement; a sensor system, wherein the arrangement is configured to: receive a material batch and provide the material batch as a material batch layer in the transporting arrangement, wherein the material batch layer comprises fragmented TLF-feed material of one or more material compositions, wherein any individual TLF-feed material pieces of the fragmented TLF-feed material have a footprint area of at most 500 cm2; provide, by means of the sensor system, sensor data of the material batch layer in the detection zone; determine, based on said sensor data, fraction data indicative of at least a first characteristic of the fragmented TLF-feed material in the material batch layer, wherein said at least a first characteristic includes material composition and/or average color and/or color distribution. The arrangement may comprise one or more elements, and/or one or more arrangements, and/or one or more systems as described in the context of the first aspect of the disclosure or any embodiments thereof.

The arrangement according to the second aspect, and any embodiments thereof, enables or facilitates providing a more homogeneous material flow. Further, the risk of any pieces of fragmented TLF-feed material in the material batch layer randomly obscuring one another or being randomly folded in some manner is further reduced. Thereby, the sensor system may provide more reliable sensor data of the material batch layer so that the at least a first characteristic of TLF-feed material in the material batch more be determined more accurately. Moreover, the arrangement advantageously enables continuous quality control monitoring. Moreover, the arrangement advantageously facilitates continuous monitoring of at least a first characteristic of TLF-feed material in the material batch. Thus, the arrangement may advantageously provide more accurate determining of said at least a first characteristic, since it is based on more data regarding the TLF-feed material in the material batch and is not extrapolated from a sample of the TLF-feed material in the material batch pre- or post-processing the material batch.

According to one embodiment, the arrangement comprises a divider arrangement configured to: receive one or more TLF-feed material items, and divide the TLF-feed material items into fragmented TLF-feed material before said material batch is provided as a material batch layer, wherein optionally the divider arrangement is configured to: divide the TLF-feed material items into fragmented TLF-feed material by cutting and/or shredding and/or tearing the TLF-feed material items. By this, the material batch layer comprising fragmented TLF-feed material may be more homogenous, thus facilitating for sensor system to make more accurate sensor data, which in turn enables more accurate determining of said at least a first characteristic of TLF-feed material in the material batch layer.

According to one embodiment, the arrangement is adapted to provide and/or receive a continuous stream of matter including fragmented TLF-feed material. A continuous stream of matter may be provided continuously or by providing each material batch of the at least one material batch in a sequential manner.

According to one embodiment, the arrangement may further comprise a pre-collection arrangement. The pre-collection arrangement may be adapted to collect at least one material batch of matter including TLF-feed material whereafter the matter including TLF-feed material is provided as a continuous stream of matter. The pre-collection arrangement may be configured to receive at least one material batch including fragmented TLF-feed material to increase a bulk density of fragmented TLF-feed material. By increasing bulk density, the material batch including the fragmented TLF-feed material may at least partially be made more compact. An advantage of this is a decreased need of storage capacity of the arrangement and the increased quantity that may be transported and/or processed by the arrangement. The pre-collection arrangement may be adapted to collect at least one material batch of matter including fragmented TLF-feed material whereafter the matter including TLF-feed material is provided as a continuous stream of matter of fragmented TLF-feed material.

According to a third aspect, a computer program is provided. The computer program comprises instructions which, when the program is executed by a computer, cause the computer to carry out the method according to the first aspect or any embodiments thereof.

According to a fourth aspect, a computer-readable storage medium is provided. The computer-readable storage medium comprises instructions which, when executed by a computer, cause the computer to carry out the method according to the first aspect or any embodiments thereof.

Effects and features of the second and third and fourth aspects are largely analogous to those described above in connection with the first aspect. Embodiments mentioned in relation to the first aspect are largely compatible with the second and third and fourth aspects. It is further noted that the inventive concepts relate to all possible combinations of features unless explicitly stated otherwise.

The invention is defined by the appended independent claims, with embodiments being set forth in the appended dependent claims, in the following description and in the drawings. It is to be understood that this disclosure is not limited to the particular component parts of the device described or steps of the methods described as such device and method may vary. It is also to be understood that the terminology used herein is for purpose of describing particular embodiments only, and is not intended to be limiting. It must be noted that, as used in the specification and the appended claims, the articles "a", "an", "the", and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings do not exclude other elements or steps.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise.

### Brief description of the drawings

The aspects of the present inventive concept, including its particular features and advantages, will be readily understood from the following detailed description and the accompanying drawings. The figures are provided to illustrate the general structures of the present inventive concept.
Fig. 1 illustrates a flow chart of different steps in a method for sorting material batch including fragmented TLF-feed material according to one embodiment of the invention;
Fig. 2a illustrates a side view of an arrangement for quality control of a material batch including fragmented TLF-feed material according to one embodiment of the invention;
Fig. 2b illustrates a top view of an arrangement for quality control of a material batch including fragmented TLF-feed material according to one embodiment of the invention;
Fig. 3 illustrates a perspective view of an arrangement for quality control of a material batch including fragmented TLF-feed material according to one embodiment of the invention;
Fig. 4a illustrates a top view of an arrangement for quality control of a material batch including fragmented TLF-feed material according to one embodiment of the invention;
Fig. 4b illustrates a top view of fragmented TLF-feed material subjected to quality control of an arrangement according to one embodiment of the invention;
Fig. 5 illustrates a perspective view of an arrangement according to one embodiment of the invention.

All figures are schematic, not necessarily to scale, and generally only show parts which are necessary in order to elucidate the disclosure, wherein other parts may be omitted or merely suggested. Throughout the figures, the same reference signs designate the same, or essentially the same features.

### Detailed description of the drawings

In the following description, the present inventive concept is described with reference to the accompanying drawings, in which currently preferred variants of the inventive concept are shown. This inventive concept may, however, be implemented in many different forms and should not be construed as limited to the variants set forth herein; rather, these variants are provided for thoroughness and completeness, and fully convey the scope of the present inventive concept to the skilled person.

Features illustrated in the attached drawings or described in the following as part of one embodiment may be used with another embodiment to yield still a further embodiment. In the interest of clarity, not all features of an actual implementation may be described in this specification. Various structures, arrangements and systems are schematically depicted in the drawings for purposes of explanation only and so as to not obscure the description with details that are well known to those skilled in the art.

The words and phrases used herein should be understood and interpreted to have a meaning consistent with the understanding of those words and phrases by those skilled in the relevant art. No special definition of a term or phrase, i.e., a definition that is different from the ordinary and customary meaning as understood by those skilled in the art, is intended to be implied by consistent usage of the term or phrase herein. To the extent that a term or phrase is intended to have a special meaning, i.e., a meaning other than that understood by skilled artisans, such a special definition is included herein in a definitional manner that directly and unequivocally provides the special definition for the term or phrase.

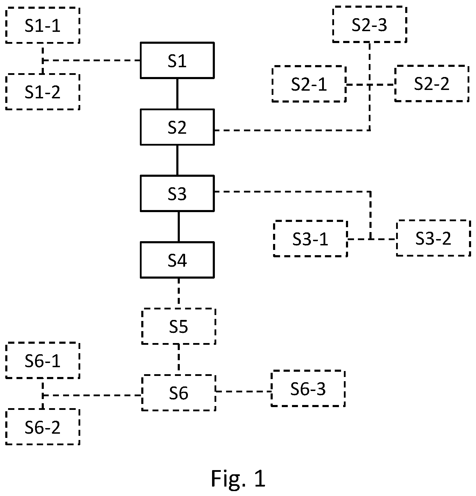

Fig. 1 illustrates a flow chart of different steps in a method of quality control of TLF-feed material of a material batch. The method may be implemented for use in a processing plant, such as a processing plant configured to process TLF-feed material waste.

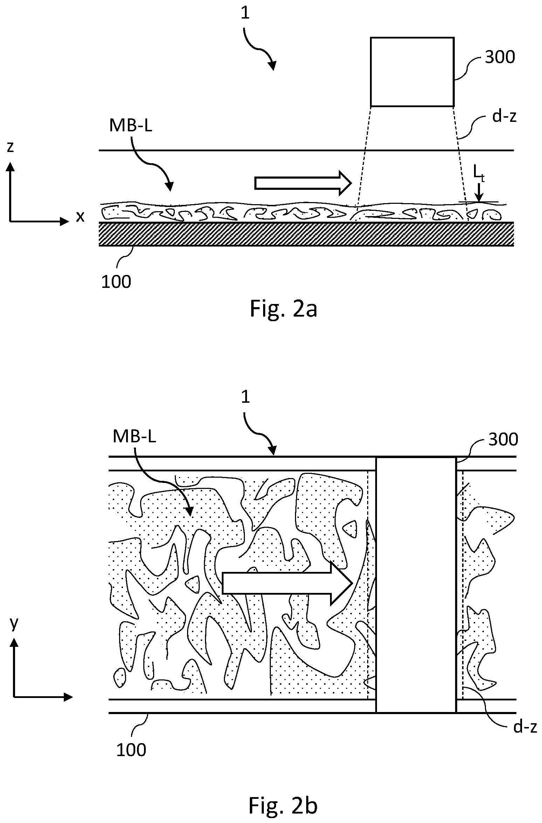

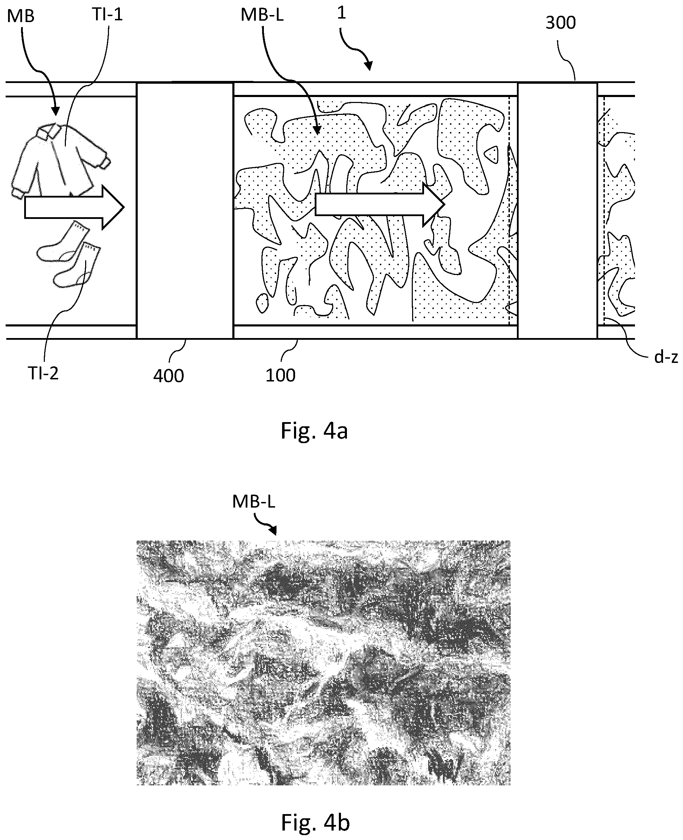

The method comprises a step S1 of providing a material batch MB as a material batch layer MB-L in a transporting arrangement 100 of a arrangement 1, as shown in Figs. 2a-2b. The material batch layer MB-L comprises fragmented TLF-feed material of one or more material compositions. As a non-limiting example, said fragmented TLF-feed material may comprise a first material composition including a first TLF-feed material constituent of cotton and a second TLF-feed material constituent of polyester. Any individual TLF-feed material pieces of at least a main portion of the fragmented TLF-feed material have a footprint area of at most 500 cm². The main portion of the fragmented TLF-feed material may be a major portion of the fragmented TLF-feed material, i.e., correspond to at least 50% of the fragmented TLF-feed material by weight or by volume. The step S1 of providing a material batch MB as a material batch layer MB-L may comprise: a step S1-1 of providing one or more TLF-feed material items; a step S1-2 of dividing, by means of a divider arrangement 400, as shown in Fig. 4a, the TLF-feed material items into fragmented TLF-feed material before said material batch MB is provided as a material batch layer MB-L. Optionally, said step S1-2 of dividing the TLF-feed material items into fragmented TLF-feed material includes cutting and/or shredding and/or tearing the TLF-feed material items.

The material batch layer MB-L comprises at least 70 WT% or 70 V/V% fragmented TLF-feed material, optionally the material batch MB comprises fragmented TLF-feed material in an interval 70-80 WT% or V/V%, 80-90 WT% or V/V%, 90-99 WT% or V/V%, or 100 WT% or V/V%. The material batch layer MB-L may include one or more material compositions at least partially or wholly selected from a group comprising: cotton, polyester, polyamides, acrylics, viscose, wool, silk, elastane, polypropylene, polyurethane, metals, acetate, cellulosic fibers, and/or combinations thereof. As a non-limiting example, the material batch layer may comprise wool as shown in Fig. 4b.

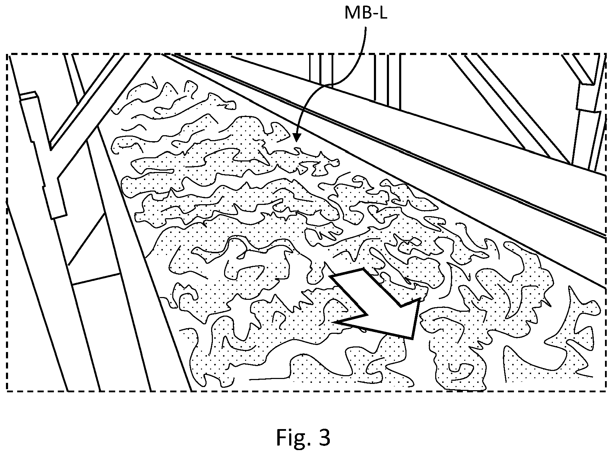

With reference to Fig. 1 and Figs. 2a-b, the method comprises a step S2 of transporting, by means of the transport arrangement 100, said material batch layer MB-L to a detection zone d-z of a sensor system 300. The material batch layer MB-L may be transported by means of a conveyor arrangement 100 as exemplified in Figs. 2a-2b, and said step of transporting, by means of the transport arrangement 100, said material batch layer MB-L to a detection zone d-z of a sensor system 300 includes a step S2-1 of transporting the material batch layer MB-L substantially across a width of a conveyor belt of the conveyor arrangement 100. Alternatively, or in combination, the material batch layer BM-L is at least partially transported in a free-falling arrangement, wherein said step of transporting, by means of the transport arrangement 100, said material batch layer MB-L to a detection zone d-z of a sensor system 300 includes: a step S2-2 of transporting the material batch layer MB-L substantially across a width of a free-falling zone of the free-falling arrangement. Moreover, said step S2 of transporting, by means of the transport arrangement 100, said material batch layer MB-L to a detection zone d-z of a sensor system 300 may further comprise: a step S2-3 of providing the material batch layer to the detection zone at a transport speed selected from a transport speed interval of 0.1 to 20 meters per second, preferably 2 to 5 meters per second. As a non-limiting example, fragmented TLF-feed material may be transported by a conveyor arrangement as illustrated in Fig. 3.

As a non-limiting example, the transport arrangement 100 may be adapted to provide the material batch layer MB-L so as to extend in a length direction x, in a width direction y, and in a height direction z of the transport arrangement 100. The transport arrangement 100 may be adapted so as to be able to transport a material batch layer MB-L having a width in an interval of 0.5-1 m, 1-1.5 m, 1.5-2 m, 2-2.5 m, 2.5-3 m, 3-3.5 m, 3.5-4 m, 4-4.5 m, 4.5-5 m, or more than 5 m, or in an interval formed by any combination thereof. The method may be adapted so as to provide a material batch layer with a layer thickness Lt in an interval 0.01-0.02 m, 0.02-0.03 m, 0.03-0.04 m, 0.04-0.05 m, 0.05-0.06 m, 0.06-0.07 m, 0.07-0.08 m, 0.08-0.09 m, 0.1-0.2 m, 0.2-0.3 m, 0.3-0.4 m, or more. The layer thickness is preferably such that the material batch layer is on average substantially homogeneous in a depth direction and/or the sensor system is capable of providing sensor data to a majority of the material batch layer in a depth direction.

With reference to Fig. 1 and Figs. 2a-b, the method comprises a step S3 of providing, by means of the sensor system 300, sensor data of the material batch layer MB-L in the detection zone d-z. Said step S3 of providing, by means of the sensor system 300, sensor data of the material batch layer MB-L may include a step S3-1 of providing, by means of a spectroscopy system, spectroscopy sensor data of the material batch layer MB-L. The spectroscopy system is preferably a near-infrared, NIR, spectroscopy system. Said step S3 of providing, by means of the sensor system 300, sensor data of the material batch layer MB-L may include a step S3-2 of providing, by means of the optical sensor system, optical sensor data of the material batch layer MB-L, wherein, optionally, the optical sensor system is configured with an artificial neural network configured to detect said at least first characteristic of fragmented TLF-feed material in the material batch MB based on images or image data acquired by the optical sensor system.

The method comprises a step S4 of determining, based on said sensor data, fraction data indicative of at least a first characteristic of the fragmented TLF-feed material in the material batch layer MB-L, wherein said at least a first characteristic includes material composition and/or average color and/or color distribution. Said fraction data may indicate at least a second characteristic of the fragmented TLF-feed material in the material batch layer MB-L, wherein said at least a second characteristic includes material composition and/or average color and/or color distribution.

As a non-limiting example, the material batch layer may comprise fragmented TLF-feed material including cotton and polyester. The method may thereby be used with a processing arrangement configured to process such a material batch layer to determine fraction data wherein a first characteristic indicates a fraction of cotton in the fragmented TLF-feed material and a second characteristic indicates a fraction of polyester in the fragmented TLF-feed material. The fraction of cotton and the fraction of polyester may be continuously determined and optionally outputted to a display device to allow a user to monitor the respective fractions. The fractions may be momentaneous values corresponding to the respective fractions of cotton and polyester in the fragmented TLF-feed material of the material batch layer currently within the detection zone. Alternatively, or in combination, the fractions may be aggregated so as to indicate fractions of cotton and polyester of the total of fragmented TLF-feed material in the material batch layer which has been conveyed through the detection zone and optionally is in the detection zone. Thereby, the respective fractions of TLF-feed material in a material batch may be determined in an accurate and convenient manner. From said fractions, and with volume date of said material batch layer, a total amounts of respective TLF-feed material may be determined.

The method may comprise a step S5 of measuring a layer thickness of the material batch layer MB-L by means of a laser triangulation system. The laser triangulation system may be comprised in the sensor system or be connected thereto. This may facilitate estimating a total volume of the material batch layer. Height information may be combined with a length and a width of the material batch layer so as to provide a volume estimate.

The method may comprise a step S6 of outputting quality data including at least a minimum and/or a maximum and/or an average and/or a median of one of the following based on said fraction data: a respective weight; a respective WT%; a respective V/V%, for each of said at least a first characteristic of the fragmented TLF-feed material, wherein, optionally, said outputting quality data includes a step S6-1 of displaying the quality data on a display device and/or provided to a computer-readable storage medium.

The method may further comprise a step S6-2 of estimating weight percentages and/or volume percentages of fragmented TLF-feed material of at least one of said at least first characteristic based on said fraction data and respective sizes of the one or more local surface areas of the material batch layer MB-L corresponding to said fraction data, and/or a step S6-3 of estimating weight and/or volume of fragmented TLF-feed material of at least one of said at least first characteristic based on said fraction data and respective sizes of the one or more local surface areas of the material batch layer MB-L associated with said fraction data, layer thickness of said one or more local surface areas of the material batch layer MB-L, and densities of said fragmented TLF-feed material of at least one of said at least first characteristic.

The arrangement 1 for quality control of TLF-feed material comprises: a transporting arrangement 100; a sensor system 300. The arrangement 1 is configured to: receive a material batch MB and provide the material batch MB as a material batch layer MB-L in the transporting arrangement 100. The material batch layer MB-L comprises fragmented TLF-feed material of one or more material compositions, wherein any individual TLF-feed material pieces of the fragmented TLF-feed material have a footprint area of at most 500 cm². The arrangement is configured to provide, by means of the sensor system 300, sensor data of the material batch layer MB-L in the detection zone d-z. The arrangement 1 is configured to determine, based on said sensor data, fraction data indicative of at least a first characteristic of the fragmented TLF-feed material in the material batch layer MB-L, wherein said at least a first characteristic includes material composition and/or average color and/or color distribution.

The arrangement 1 may comprise a divider arrangement 400 configured to: receive one or more TLF-feed material items TI-1, and divide the TLF-feed material items TI-1, TI-2 into fragmented TLF-feed material before said material batch MB is provided as a material batch layer MB-L. Optionally, the divider arrangement 400 is configured to: divide the TLF-feed material items TI-1, TI-2 into fragmented TLF-feed material by cutting and/or shredding and/or tearing the TLF-feed material items TI-1, TI-2.

Fig. 3 illustrates a perspective view of an arrangement for quality control of a material batch including fragmented TLF-feed material according to one embodiment of the invention. Fig. 4b illustrates a top view of fragmented TLF-feed material subjected to quality control of an arrangement according to one embodiment of the invention;

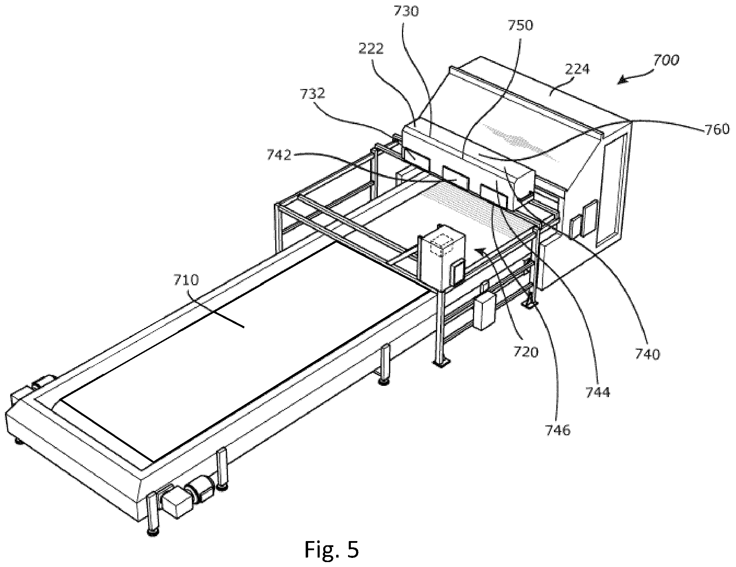

Fig. 5 illustrates a perspective schematic view of a processing arrangement 700. The processing arrangement 700 may comprise one or more of the features described below.

The processing arrangement 700 is fed with a material batch including TLF-feed material. The material batch is provided as a material batch layer which is conveyed through a detection zone 720. However, the material batch layer may be provided through the detection zone by any suitable means or manually without any technical means. A light source arrangement 730 and a NIR spectroscopy system 222 are provided. The spectroscopy system is adapted to receive and analyze 732, from the light source arrangement 730, which is reflected and/or scattered by the pieces light source arrangement 730, which is reflected and/or scattered by the material batch layer in the detection zone 720. Hence, the NIR spectroscopy system 222 typically acquires a spectrum of the material batch layer that is conveyed through the detection zone 720. The NIR spectroscopy system 222 of the classification and sorting station 700 is configured to discriminate portions of the material batch layer based on the acquired spectrum, wherefrom characteristics of TLF-feed material in the material batch layer may be provided. In other words, the NIR system 222 is typically set up such that at least a first specific characteristic of TLF-feed material of the material batch layer is determined.

The processing arrangement may further comprise a spectroscopy system 760 configured to acquire a spectrum of the TLF-feed material in the material batch layer that is conveyed through the detection zone 720. The spectroscopy system 760 may through the acquired spectrum determine the different characteristics of the TLF-feed material that is conveyed through the detection zone 720.

The processing arrangement 700 may further comprise a laser triangulation system 740 configured to determine height information of the material batch layer that is conveyed through the detection zone 720. The laser triangulation system 740 is typically configured to emit a line of laser light 742 towards the detection zone 720. The depicted laser triangulation system 740 includes a camera-based sensor arrangement 744 configured to receive and analyze light 746 which is reflected and/or scattered by the material batch layer in the detection zone 720. By means of the laser triangulation system 740 the processing arrangement 700 may be able to detect TLF-feed material in the material batch layer that the NIR spectroscopy system 222 have difficulties to detect. By detecting height differences on a conveyor belt used to convey the material batch layer being processed, the processing arrangement may combine such height information with the acquired information from the NIR spectroscopy system 222 to determine if there is any TLF-feed material in the material batch layer that is hard to detect on the conveyor belt. Accordingly, further characteristics of TLF-feed material may be determined.

The processing arrangement 700 may further comprise a camera 750 configured to acquire images of TLF-feed material in the material batch layer that is conveyed through the detection zone 720. The processing arrangement 700 may comprise an artificial neural network in combination with the camera 750. Such artificial neural network may be configured to detect different characteristics of TLF-feed material in the material batch layer that is conveyed through the detection zone 720 based on images acquired by the camera 750. In other words, characteristics of TLF-feed material in the material batch layer may thus be determined by the artificial neural network from the, by the camera, acquired images. The characteristics may be a TLF-feed material composition, shape, a color, features at the surface or anything in the visual appearance of the TLF-feed material in the material batch layer that may be determined and classified by the artificial neural network. The camera 750 may provide the possibility to determine further characteristics of TLF-feed material in the material batch layer. With the help of the artificial neural network it may be possible to determine characteristics of TLF-feed material of the same material composition depending on quality and origin.

While the foregoing is directed to embodiments of the disclosure, other and further embodiments may be devised without parting from the inventive concept discussed herein.

## Claims

1. Method of quality control of textile, leather, and/or footwear feed material, TLF-feed material, the method comprising:
- providing at least one material batch (MB) as a material batch layer (MB-L) in a transporting arrangement (100), wherein the material batch layer (MB-L) comprises fragmented TLF-feed material of one or more material compositions, wherein any individual pieces of at least a main portion of the fragmented TLF-feed material have a footprint area of at most 500 cm²;
- transporting, by means of the transport arrangement (100), said material batch layer (MB-L) to a detection zone (d-z) of a sensor system (300);
- providing, by means of the sensor system (300), sensor data of the material batch layer (MB-L) in the detection zone (d-z);
- determining, based on said sensor data, fraction data indicative of at least a first characteristic of the fragmented TLF-feed material in the material batch layer (MB-L), wherein said at least a first characteristic includes material composition and/or average color and/or color distribution.

2. Method according to claim 1, wherein said step of providing said at least one material batch (MB) as a material batch layer (MB-L) further comprises:
- providing one or more items (TI-1, TI-2) of TLF-feed material;
- dividing, by means of a divider arrangement (400), the TLF-feed material items (TI-1, TI-2) into fragmented TLF-feed material before said material batch (MB) is provided as a material batch layer (MB-L),
- wherein, optionally, said step of dividing the TLF-feed material items (TI-1, TI-2) into fragmented TLF-feed material includes cutting and/or shredding and/or tearing the TLF-feed material items (TI-1, TI-2).

3. Method according to any of the preceding claims, wherein said fraction data indicates at least a second characteristic of the fragmented TLF-feed material in the material batch layer (MB-L), wherein said at least a second characteristic includes material composition and/or average color and/or color distribution.

4. Method according to any of the preceding claims, wherein the material batch layer (MB-L) is at least partially transported by means of a conveyor arrangement (100), wherein said step of transporting, by means of the transport arrangement (100), said material batch layer (MB-L) to a detection zone (d-z) of a sensor system (300) includes:
- transporting the material batch layer (MB-L) substantially across a width of a conveyor belt of the conveyor arrangement (100).

5. Method according to any of the preceding claims, wherein the material batch layer (BM-L) is at least partially transported in a free-falling arrangement, wherein said step of transporting, by means of the transport arrangement (100), said material batch layer (MB-L) to a detection zone (d-z) of a sensor system (300) includes:
- transporting the material batch layer (MB-L) substantially across a width of a free-falling zone of the free-falling arrangement.

6. Method according to any of the preceding claims, wherein said step of providing, by means of the sensor system (300), sensor data of the material batch layer (MB-L) includes:
- providing, by means of a spectroscopy system, spectroscopy sensor data of the material batch layer (MB-L),
- wherein the spectroscopy system is preferably a near-infrared, NIR, spectroscopy system.

7. Method according to any of the preceding claims, wherein said step of providing, by means of the sensor system (300), sensor data of the material batch layer (MB-L) includes:
- providing, by means of the optical sensor system, optical sensor data of the material batch layer (MB-L),
- wherein, optionally, the optical sensor system is configured with an artificial neural network configured to detect said at least first characteristic of fragmented TLF-feed material in the material batch (MB) based on images or image data of the optical sensor data acquired by the optical sensor system.

8. Method according to any of the preceding claims, wherein the material batch layer (BM-L) comprises at least 70 WT% or 70 V/V% fragmented TLF-feed material, optionally the material batch (MB) comprises fragmented TLF-feed material in an interval 70-80 WT% or V/V%, 80-90 WT% or V/V%, 90-99 WT% or V/V%, or 100 WT% or V/V%.

9. Method according to any of the preceding claims, wherein the material batch layer (MB-L) includes one or more material compositions at least partially or wholly selected from a group comprising: cotton, polyester, polyamides, acrylics, viscose, wool, silk, elastane, polypropylene, polyurethane, metals, acetate, cellulosic fibers, and/or combinations thereof.

10. Method according to any of the preceding claims, wherein said step of transporting, by means of the transport arrangement (100), said material batch layer (MB-L) to a detection zone (d-z) of a sensor system (300) comprises:
- providing the material batch layer to the detection zone at a transport speed selected from a transport speed interval of 0.1 to 20 meters per second, preferably 2 to 5 meters per second.

11. Method according to any of the preceding claims, comprising:
- measuring a layer thickness of the material batch layer (MB-L) by means of a laser triangulation system.

12. Method according to any of the preceding claims, comprising:
- outputting quality data including at least a minimum and/or a maximum and/or an average and/or a median of one of the following based on said fraction data:
∘ a respective weight;
∘ a respective WT%;
∘ a respective V/V%,
for each of said at least a first characteristic of the fragmented TLF-feed material,
- wherein, optionally, said outputting quality data includes displaying the quality data on a display device and/or provided to a computer-readable storage medium.

13. Method according to any of the preceding claims, further comprising:
- estimating weight percentages and/or volume percentages of fragmented TLF-feed material of at least one of said at least first characteristic based on said fraction data and respective sizes of the one or more local surface areas of the material batch layer (MB-L) corresponding to said fraction data, and/or
- estimating weight and/or volume of fragmented TLF-feed material of at least one of said at least first characteristic based on said fraction data and respective sizes of the one or more local surface areas of the material batch layer (MB-L) associated with said fraction data, layer thickness of said one or more local surface areas of the material batch layer (MB-L), and densities of said fragmented TLF-feed material of at least one of said at least first characteristic.

14. Arrangement (1) for quality control of textile, leather, and/or footwear feed material, TLF-feed material, the arrangement (1) comprising:
- a transporting arrangement (100);
- a sensor system (300),
wherein the arrangement (1) is configured to:
- receive a material batch (MB) and provide the material batch (MB) as a material batch layer (MB-L) in the transporting arrangement (100), wherein the material batch layer (MB-L) comprises fragmented TLF-feed material of one or more material compositions, wherein any individual TLF-feed material pieces of the fragmented TLF-feed material have a footprint area of at most 500 cm²;
- provide, by means of the sensor system (300), sensor data of the material batch layer (MB-L) in the detection zone (d-z);
- determine, based on said sensor data, fraction data indicative of at least a first characteristic of the fragmented TLF-feed material in the material batch layer (MB-L), wherein said at least a first characteristic includes material composition and/or average color and/or color distribution.

15. Arrangement (1) according to claim 14, further comprising:
- a divider arrangement (400) configured to:
∘ receive one or more TLF-feed material items (TI-1), and
∘ divide the TLF-feed material items (TI-1, TI-2) into fragmented TLF-feed material before said material batch (MB) is provided as a material batch layer (MB-L),
- wherein optionally the divider arrangement (400) is configured to:
∘ divide the TLF-feed material items (TI-1, TI-2) into fragmented TLF-feed material by cutting and/or shredding and/or tearing the TLF-feed material items (TI-1, TI-2).
